# FASCICULE DE BREVET EUROPEEN

(11) **EP 3 334 328 B1**
(45) Date de publication et mention de la délivrance du brevet: **17.02.2021**
(21) Numéro de dépôt: 16757325.2
(22) Date de dépôt: 04.08.2016
(51) Int. Cl.: A61B 3/032, A61B 3/00

(54) **PROCÉDÉ DE DÉTERMINATION DE MOYENS D'AIDE VISUELLE PAR RAPPORT AU COMPORTEMENT D'UN INDIVIDU SOUMIS A UN TEST**
VERFAHREN ZUR BESTIMMUNG VISUELLER SEHHILFEN MIT BEZUG AUF DAS VERHALTEN EINER EINEM TEST UNTERZOGENEN EINZELPERSON
METHOD FOR DETERMINING VISUAL AID MEANS BY REFERENCE TO THE BEHAVIOUR OF AN INDIVIDUAL SUBJECTED TO A TEST

(30) Priorité: 14.08.2015 FR 1557734
(43) Date de publication de la demande: 20.06.2018
(73) Titulaire: Essilor International, 94220 Charenton-Le-Pont (FR)
(72) Inventeur: SCHERLEN, Anne-Catherine, 94227 Charenton-Le-Pont (FR); FAURE, Géraldine, 13001 Marseille (FR); GOLDSCHMIDT, Miryam, 1009 Pully (CH)
(74) Mandataire: Ipsilon
(86) Numéro de dépôt international: PCT/FR2016/052026
(87) Numéro de publication internationale: WO 2017/029442

(56) Documents cités:
- EP-A1- 2 168 473
- WO-A1-2015/024790
- US-A1- 2006 078 858
- US-A1- 2014 285 769

## Description

L'invention se rapporte à un procédé de détermination de moyens d'aide visuelle par rapport au comportement d'un individu soumis à un test.

Plus spécifiquement, l'invention concerne la mise en place et l'exploitation d'un test spécifique pour évaluer les capacités oculomotrices d'un individu ayant une perte du champ de vision central, défini par une anomalie visuelle de type scotome central dû par exemple à une dégénérescence maculaire liée à l'âge (ou DMLA). En effet, la perte ou la fragilité de la vision centrale altère la commande oculomotrice, qu'il est important de pouvoir détecter afin d'y remédier avec des moyens de corrections appropriés. Cette commande oculomotrice peut se définir à partir de paramètres liés aux mouvements des yeux tels que par exemple, des saccades, des fixations, une stabilité oculaire.

L'évaluation des capacités sensorielles et oculomotrices d'un individu, constitue une donnée indispensable pour comprendre et caractériser les difficultés de lecture et d'exploration de scène du sujet. Elle permet également de déterminer et de consolider une aide à la décision pour la prise en charge des sujets : détermination et adaptation des moyens d'aides visuelles optiques et/ou électronique, et/ou la mise en place d'une rééducation visuelle, afin d'obtenir une réhabilitation oculomotrice. Le document US 2006/078858 A1 représente l'état de la technique le plus proche décrit un procédé et un système de détermination d'une aide visuelle suite au comportement d'un individu lors d'une phase de lecture spécifique standard en présentant à l'individu une succession de mots usuels et en attribuant un indicateur optique lié aux erreurs de lecture relevées et permettant de caractériser la qualité de la lecture du texte.

Un procédé de détermination de moyens d'aide visuelle selon l'invention peut être utilisé par tous les professionnels de santé, tels que par exemple, les opticiens, les ophtalmologistes, les orthoptistes, et les médecins, pour diagnostiquer les capacités de lecture des individus, se traduisant essentiellement par une oculomotricité, mais aussi pour aider les individus à prendre conscience de leurs anomalies visuelles et déterminer les moyens d'aide visuelle les plus adaptés au profil sensoriel moteur desdits individus.

L'invention a pour objet un procédé de détermination de moyens d'aide visuelle suite au comportement d'un individu lors d'une phase de lecture spécifique.

Pour la suite de la description, le terme « test de lecture classique » correspond à une lecture d'un texte composé de mots usuels et ayant un sens intelligible.

La principale caractéristique d'un procédé selon l'invention est qu'il comprend les étapes suivantes,
- une étape de soumission à l'individu d'un test spécifique de lecture d'un support comportant un texte comprenant une succession de mots usuels et dans lesquelles ont été introduits des défauts entre lesdits mots à des emplacements prédéterminés,
- une étape de lecture à voix haute dudit texte par l'individu,
- une étape d'attribution d'un indicateur optique lié aux erreurs de lecture relevées et permettant de caractériser la qualité de la lecture,
- une étape de sélection des moyens d'aide visuelle les plus appropriés en fonction de l'indicateur optique déterminé.

Ce procédé vise à mettre en évidence une anomalie visuelle à travers la lecture d'un texte doté de défauts, puis à proposer des moyens de correction les plus appropriés pour remédier au moins partiellement à cette anomalie afin de proposer à l'individu des conditions de lecture satisfaisantes. Ce type de procédé est particulièrement adapté à la détection d'un scotome central, lié par exemple à une DMLA, dont la présence va masquer des zones particulières du texte et engendrer une lecture biaisée. Un exemple de défaut introduit dans le texte peut être constitué par l'insertion d'une lettre isolée entre deux mots. Le scotome peut alors masquer, soit cette lettre isolée, soit au moins une partie de l'un des deux mots encadrant cette lettre isolée. Le terme « texte » est général, et peut inclure par exemple des séquences de mots usuels entrecoupées de défauts, des motifs facilement identifiables et répétitifs, des idéogrammes suivant les langues considérées. Tous ces éléments constitutifs du texte peuvent être présentés avec des tailles constantes et/ou variables, et/ou avec un ordonnancement particulier en lignes et/ou en colonnes, et/ou avec des couleurs variées. L'individu est sensé déchiffrer visuellement les éléments constitutifs du texte. L'indicateur optique, qui peut par exemple être matérialisé par un chiffre associé ou non à une lettre, est représentatif d'une anomalie visuelle et de son amplitude. Cet indicateur est issu de l'étape du test de lecture du texte, et est fonction du nombre d'erreurs de lecture relevées et de la taille du texte sur lequel sont produites lesdites erreurs. Il est supposé qu'une correspondance a été préalablement établie entre l'indice optique et une anomalie visuelle. Cette correspondance peut par exemple être disponible à travers une table établissant une corrélation entre l'indice optique et une anomalie visuelle. Les moyens d'aide visuelle peuvent par exemple être matérialisés par un équipement spécifique de type loupes, et ont pour but de corriger au moins partiellement l'anomalie visuelle détectée chez l'individu, afin qu'il puisse retrouver des conditions de lecture acceptables lui permettant de comprendre un texte standard. Une base de données listant par exemple des moyens d'aide à la lecture appropriés, en fonction d'une anomalie visuelle particulière, peut être avantageusement utilisée dans le cadre d'un procédé de détermination selon l'invention.

Avantageusement, chaque défaut de texte est à choisir parmi un ajout d'au moins un blanc, un ajout d'au moins une lettre, une suppression d'au moins un blanc et une suppression d'au moins une lettre. Ces défauts demeurent de dimensions minimes par rapport aux dimensions des mots usuels constituant le texte.

De façon préférentielle, le texte comprend plusieurs paragraphes comprenant des lettres d'une taille spécifique et un espacement spécifique entre les lettres de chaque mot et entre les mots. De cette manière, la présence de plusieurs paragraphes ayant des mots de tailles différentes, permet de mieux cerner les caractéristiques de l'anomalie visuelle de l'individu.

Préférentiellement, tous les paragraphes du support ont des tailles de lettres différentes, et des espacements différents entre les lettres de chaque mot et entre les mots.

De façon avantageuse, l'indicateur optique est fonction des caractéristiques dimensionnelles des paragraphes dans lesquels ont été commises les erreurs de lecture. L'indicateur optique tient compte, non seulement du nombre d'erreurs commises lors de la lecture du texte, mais également de la taille des lettres et des mots sur lesquels elles ont été commises.

Avantageusement, le support de lecture comprend des séquences de motifs identifiables, caractérisées par une succession de groupes de motifs possédant chacun un nombre spécifique de motifs et séparés les uns des autres par des blancs, l'espacement entre deux motifs successifs dans un groupe étant identique pour tous les groupes. Ces motifs ne doivent pas avoir un contour et/ou une géométrie trop complexe, sous peine de ne pas pouvoir être identifiés par l'individu. De cette manière, chaque motif peut par exemple être carré, rond, rectangulaire, ovale ou en losange, ou résulter d'une combinaison de ces formes.

De façon préférentielle, les tailles des blancs entrecoupant les groupes de motifs sont différentes.

Préférentiellement, chaque séquence comprend des motifs identiques.

De façon avantageuse, l'indicateur optique est apte à révéler une anomalie visuelle constituée par un scotome et/ou une instabilité oculaire. Une instabilité oculaire peut émaner de la présence d'un scotome, ou être provoquée sans cause identifiable particulière.

Avantageusement, l'individu adopte une posture naturelle durant l'étape de lecture du support. De cette manière, un procédé selon l'invention est ergonomique et souple, car il permet d'éviter à un individu des postures contraignantes qui seraient susceptibles de le gêner et de biaiser les conditions de lecture du texte.

De façon préférentielle, le support est un écran placé à une distance prédéterminée de l'individu. Cet écran peut par exemple être celui d'un téléviseur ou d'un ordinateur.

Selon un autre mode de réalisation préféré d'un procédé selon l'invention, le support est un dispositif manuel que l'individu manipule lors de la réalisation du test de lecture. De cette manière, l'individu peut adapter à sa convenance la position du support, pour se retrouver dans une posture de lecture naturelle.

De façon avantageuse, un procédé de détermination selon l'invention, comprend un deuxième test de lecture sur un support présentant au moins une couleur. En effet, afin d'affiner la détermination de l'anomalie visuelle, il peut être procédé à des tests de lecture et/ou visualisation simples et complémentaires. Ces tests complémentaires doivent être rapides et simples à mettre en œuvre pour ne pas rallonger significativement un procédé de détermination selon l'invention.

Avantageusement, un procédé de détermination selon l'invention, comprend un troisième test de lecture sur un support présentant une cible mobile dont la trajectoire est suivie visuellement par l'individu. En effet, afin d'affiner la détermination de l'anomalie visuelle, il peut être procédé à d'autres tests de lecture et/ou visualisation simples et complémentaires.

De façon préférentielle, un procédé de détermination selon l'invention, comprend une étape préalable de lecture par l'individu d'un texte ayant un sens et utilisant des mots usuels, les erreurs de lecture relevées lors de cette étape, vont conditionner les conditions de réalisation de l'étape de soumission du test spécifique. En effet, si l'individu commet déjà des erreurs de lecture lors de ce test de lecture préliminaire, il pourra être proposé à l'individu de lire directement une zone particulière du texte avec des défauts, correspondant à une taille particulière de lettre et/ou de défauts.

Préférentiellement, un procédé de détermination selon l'invention, comprend une étape de réalisation d'une table de critères optiques, comprenant au moins trois catégories distinctes et identifiables, une première catégorie correspondant à une vision normale, une deuxième catégorie pour laquelle la vision présente quelques anomalies, et une troisième catégorie pour laquelle la vision présente un nombre important d'anomalies. En fonction de la finesse de détection de l'anomalie visuelle recherchée, des subdivisions de catégories peuvent être envisagées.

De façon avantageuse, chaque catégorie est repérée par un chiffre et/ou une lettre qui est fonction des caractéristiques des anomalies visuelles détectées.

Avantageusement, un procédé de détermination selon l'invention, comprend une étape d'enregistrement de la table de critères optiques. De cette manière, cette table est disponible à tout moment. Préférentiellement, cette table de correspondance peut s'afficher sur un écran.

De façon préférentielle, un procédé de détermination selon l'invention, comprend une étape d'élaboration d'une base de données établissant une corrélation entre l'indice optique et des moyens d'aide à une correction visuelle les plus adaptés. Cette base de données peut établir, soit une corrélation directe entre l'indice optique et les moyens d'aide à la lecture pour remédier au moins partiellement à l'anomalie visuelle, soit une corrélation entre une anomalie visuelle et lesdits moyens d'aide à la lecture.

Préférentiellement, les moyens d'aide à la vision sont à choisir parmi des loupes, des lunettes microscopiques, des systèmes télescopiques de type Galilée ou Kepler, des aides électroniques portables, des aides électroniques de type télé-agrandisseurs, des lampes, des filtres, des lunettes équipées de moyens de réalité augmentée, des verres progressifs à forte addition, et des séances de rééducation oculomotrice.

L'invention a pour autre objet un dispositif pour la réalisation d'un procédé de détermination conforme à l'invention.

La principale caractéristique d'un dispositif selon l'invention est qu'il est constitué par un terminal numérique mobile possédant un logiciel adapté et un accès à des bases de données enregistrées.

Avantageusement, le terminal numérique mobile est une tablette tactile, apte à faire apparaitre le support de lecture et à enregistrer la voix de l'individu, ladite tablette possédant un logiciel de détermination de l'indice optique ainsi qu'une table de correspondance entre ledit indice optique et l'anomalie visuelle correspondante, ladite tablette possédant en outre un accès à des bases de données établissant une corrélation entre l'indice optique et des moyens d'aide visuelle les plus adaptés.

Un procédé de détermination de moyens d'aide visuelle selon l'invention présente l'avantage de pouvoir être réalisé aussi bien en monoculaire qu'en binoculaire. Il est fréquent dans la prise en charge de la basse vision d'adapter des équipements optiques en monoculaire ou en binoculaire selon la rivalité sensorielle ou oculomotrice des deux yeux. Autrement dit, voir ou lire avec les 2 yeux peut être plus pénalisant pour un lecteur. Ainsi, une adaptation sur seulement un œil peut donner de meilleures performances. Il a de plus l'avantage d'être facile et rapide à mettre en œuvre, et de proposer à un individu d'adopter une posture naturelle de lecture. De cette manière, les résultats du procédé ne seront pas biaisés par des postures de lecture contraignantes, susceptibles de dénaturer le principe dudit procédé.

On donne ci-après, une description détaillée d'un mode de réalisation préféré d'un procédé de détermination selon l'invention, en se référant aux figures suivantes:
- La figure 1 est une vue d'un premier exemple de texte et d'une tâche matérialisant une anomalie visuelle,
- La figure 2 est une vue d'un deuxième exemple de texte et d'une tâche matérialisant une anomalie visuelle,
- Les figures 3a, 3b, 3c sont trois vues d'un même texte doté de défauts et d'une tâche matérialisant une anomalie visuelle dans trois positions différentes sur ledit texte,
- La figure 4 est un exemple d'un texte doté de défauts et adapté à la réalisation d'un procédé de détermination selon l'invention,
- La figure 5 est un diagramme comparatif du nombre d'erreurs de lecture commises sur un texte intelligible et sur un texte doté de défauts, avec ou non la présence d'une anomalie visuelle,
- La figure 6 est un exemple d'un tableau de synthèse montrant différentes catégories correspondant chacune à un comportement de lecture d'un texte intelligible et d'un texte doté de défauts.

Certains individus possèdent des anomalies visuelles, telles que par exemple un scotome et/ou une instabilité optique, qui peuvent avoir des répercussions majeures, notamment au niveau de la lecture d'un texte.

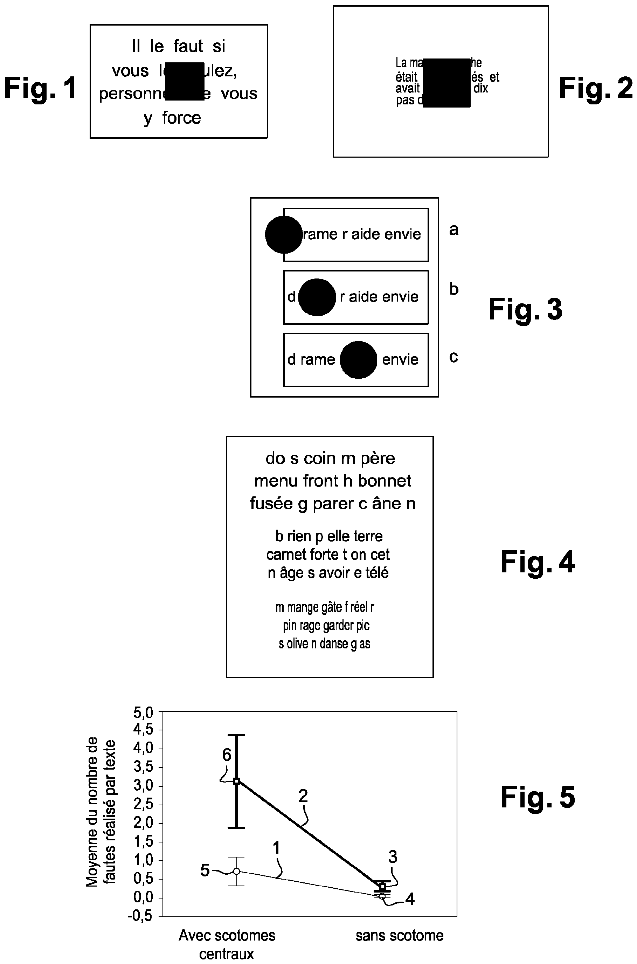

En effet, en se référant aux figures 1 et 2, la présence d'un scotome central masque des zones de lecture plus ou moins importantes, en fonction de la taille des lettres d'un texte, de l'espacement entre les mots, de l'espacement entre les lignes, etc....

En se référant à la figure 1, lorsque la taille des caractères est importante, il demeure possible de deviner les mots estropiés par la présence du scotome, mais oblige néanmoins à effectuer un effort intellectuel important qu'il semble difficile de maintenir sur la totalité d'un texte ou d'un livre.

En revanche, en se référant à la figure 2, lorsque la taille des caractères est trop petite, une lecture intelligible d'un texte demeure quasiment impossible.

De façon générale, lire avec un scotome central perturbe les processus de lecture : diminution de l'acuité visuelle, sensibilité aux contrastes et perte du référentiel moteur central. La commande oculomotrice est la plus rapide, la plus précise et la plus stable en vision centrale. La présence d'un scotome central engendre une fixation périphérique dite fixation excentrée. Cette perte de référentiel engendre entre autre, une augmentation de l'instabilité de fixation, une diminution de l'amplitude des saccades, limitant ainsi l'efficacité de lecture.

De plus, lorsqu'une personne lit avec un scotome, une partie de l'information visuelle est masquée. Elle va devoir mouvoir son œil et ainsi son scotome pour démasquer les lettres des mots pour les lire.

Malheureusement, les instabilités de l'œil, la perte des indices de la forme des mots (processus bas niveau de la reconnaissance des mots) réduit le temps de reconnaissance du texte. De plus, il est très fréquent que le patient, même s'il ne voit pas toutes les lettres des mots, suite à la difficulté à mouvoir les yeux, utilise le contexte sémantique du texte pour deviner les mots. La charge cognitive associée à cette stratégie est importante et il est très fréquent que le lecteur relate une incohérence dans sa lecture après quelques phrases lues. Une fatigue de lecture apparaît, un temps de lecture plus important et également un abandon et un découragement apparaît.

Dans l'optique d'une prise en charge efficace, il est indispensable de connaître les stratégies de lecture réelles des sujets et de déterminer la manière dont le sujet arrive à obtenir une lecture efficiente avec les contraintes de la présence d'un scotome central ou d'une perte de vision centrale.

Un procédé de détermination d'un comportement visuel selon l'invention vise à détecter une anomalie visuelle de type scotome, puis à en évaluer les capacités à mouvoir et contrôler les mouvements oculaires afin de proposer une aide visuelle la plus adaptée aux caractéristiques de cette anomalie.

Pour la description détaillée qui va suivre, l'exemple d'anomalie visuelle considéré est un scotome central.

Un procédé de détermination d'une aide visuelle suite au comportement d'un individu lors d'une phase de lecture spécifique, comprend les étapes suivantes :
A- une étape de soumission à l'individu d'un test spécifique de lecture d'un support comportant un texte comprenant une succession de mots usuels et dans lesquelles ont été introduits des défauts entre lesdits mots à des emplacements prédéterminés. De façon générale, le texte est constitué de la manière suivante,
   ∘ Successions de mots sans signification entre eux : l'individu ne peut pas s'aider du contexte pour deviner les mots,
   ∘ Utilisation de lettres isolées pour créer la confusion entre les mots: si le sujet ne voit pas l'espace entre la lettre isolée et le mot, il peut dire un autre mot, non affiché. Le choix des mots et des lettres isolées a été judicieusement fait pour pousser le sujet à faire des erreurs.
   ∘ Choix des mots qu'on peut lire sans la première ou la dernière lettre pour induire des confusions, une interprétation des fautes réalisées par le lecteur,

En se référant aux figures 3a, 3b, 3c un premier exemple d'un texte dans lequel ont été introduit des défauts peut consister en un paragraphe composé de plusieurs lignes superposées. Chaque ligne est ainsi constituée d'une succession de mots usuels, entrecoupés de défauts consistant chacun en l'insertion d'une seule lettre entre lesdits mots. Cette insertion n'est pas régulière. De cette manière, deux mots usuels peuvent se retrouver directement l'un derrière l'autre. En fonction de la position de la tâche d'occultation matérialisant le scotome au sein d'une même succession de mots dotée de défauts, la lecture sera complètement différente. Ainsi, la lecture des mots apparaissant à la figure 3a sera complètement différente de celle des mots apparaissant à la figure 3b, et différente de celle des mots apparaissant à la figure 3c. A cause de l'insertion de ces défauts dans le texte, les successions de mots sont dépourvues de sens, et l'individu atteint de cette anomalie visuelle, ne peut donc pas reconstituer intellectuellement les lettres manquantes pour tenter de donner un sens à cette succession de mots.

En se référant à la figure 4, un deuxième exemple d'un texte dans lequel ont été introduit des défauts, peut consister en plusieurs paragraphes, en l'occurrence trois paragraphes, ayant chacun des tailles de lettres différentes et un espacement entre lesdites lettres différent. Ainsi, un premier paragraphe est écrit en grosses lettres et possède trois lignes superposées, chaque ligne consistant en une succession de mots usuels entrecoupés de lettres isolées constituant les défauts introduits dans le texte. Ces lettres isolées sont chacune placées entre deux mots usuels. Toutefois, toutes les séquences de deux mots usuels successifs ne sont pas systématiquement l'objet de l'insertion d'une lettre isolée. La taille de toutes les lettres constituant ce premier paragraphe, est constante ainsi que l'espacement entre les lettres d'un mot usuel, et l'espacement entre deux mots usuels et entre une lettre isolée et un mot usuel. Un deuxième paragraphe présentant les mêmes caractéristiques de structure que le premier paragraphe est écrit avec des lettres moyennes, dont la taille est inférieure à celle des lettres du premier paragraphe. Un troisième paragraphe présentant les mêmes caractéristiques de structure que le premier et le deuxième paragraphe est écrit avec des petites lettres dont la taille est inférieure à celle des lettres du deuxième paragraphe.

Un troisième exemple d'un texte dans lequel ont été introduit des défauts, peut consister à remplacer les lettres par des motifs identifiables ayant une géométrie simple, comme par exemple carrée, rectangulaire ou en losange. De cette manière, chaque phrase apparaitrait sous forme codée, avec des séquences de motifs entrecoupées par des espacements plus ou moins importants, l'espacement entre les motifs d'une même séquence étant constant.
B- une étape de lecture à voix haute dudit texte par l'individu. Cette étape de lecture à voix haute est indispensable pour détecter les erreurs que l'individu peut commettre en lisant le texte doté de ses défauts. Selon une variante de réalisation de l'invention, cette étape de restitution peut se faire par écrit.
C- une étape de relevés de ces défauts. Cette étape peut s'effectuer automatiquement au moyen d'un appareillage apte à détecter les sons émis lors d'une lecture à voix haute et à relever instantanément et automatiquement les erreurs de lectures par comparaison avec une lecture de référence préenregistrée. Les erreurs peuvent également être relevées manuellement par une tiers personne qui a également accès au texte de lecture.
D- une étape optionnelle de lecture par l'individu d'un texte intelligible constitué de phrases avec des mots usuels et ayant un sens. Cette étape optionnelle peut intervenir à tout moment durant un procédé de détermination selon l'invention. Préférentiellement, elle constitue une étape préalable à l'étape de lecture du texte doté de défauts. En effet, cette étape optionnelle est facile et rapide à mettre en œuvre, car les erreurs de lecture faites par l'individu peuvent être détectées, même sans avoir accès au texte. Il suffit d'écouter ce qu'énonce l'individu en lisant le texte, pour savoir s'il commet beaucoup, peu ou pas d'erreurs. La figure 5 met en évidence l'importance d'un procédé de détermination selon l'invention pour révéler la présence d'un scotome par rapport à un test de lecture classique sur un texte cohérent et intelligible. La courbe 1 se rapporte à un test de lecture sur un texte possédant des défauts, et la courbe 2 se rapporte à un test de lecture sur un texte cohérent et intelligible. Les deux points de ces deux courbes situés 3,4 à droite représentent la moyenne du nombre d'erreurs de lecture commise par un individu sans scotome. Pour ces deux points, les deux tests de lecture donnent environ le même résultat à savoir moins de 0.5 fautes de lectures. Les deux points 5, 6 de ces deux courbes situés à gauche représente le nombre d'erreurs de lecture commise par un individu avec un scotome central. Pour le test de lecture sur le texte cohérent et intelligible, le nombre d'erreurs 5 de lecture demeure faible, inférieur à 1. En revanche, le nombre d'erreurs 6 de lecture sur le texte possédant des défauts est beaucoup plus important, autour de trois. Une telle différence de résultats révèle l'importance d'un procédé de détermination selon l'invention pour détecter une anomalie visuelle de type scotome central. Le test de lecture sur un texte cohérent et intelligible vient en appoint d'un procédé selon l'invention. Si, à titre d'exemple, un individu commet un nombre d'erreurs inhabituelles lors du test de lecture classique, on pourra alors lui demander de commencer le test de lecture sur le texte doté de défauts, sur un paragraphe ayant des lettres de taille moyenne ou importante.
E- une étape d'attribution d'un indicateur optique lié aux erreurs de lecture relevées et permettant de caractériser la qualité de la lecture. La qualité de lecture comprend également la capacité de lecture associée à l'interprétation. Cet indicateur est fonction du nombre de fautes de lectures, de l'emplacement des fautes selon la taille des lettres du paragraphe lu.

Il est à noter que le test de lecture sur un texte possédant des défauts, et l'analyse des fautes de lectures, permet en plus d'évaluer l'acuité de lecture de l'individu, d'évaluer ses difficultés à mouvoir son scotome, et à connaitre indirectement sa capacité oculomotrice.

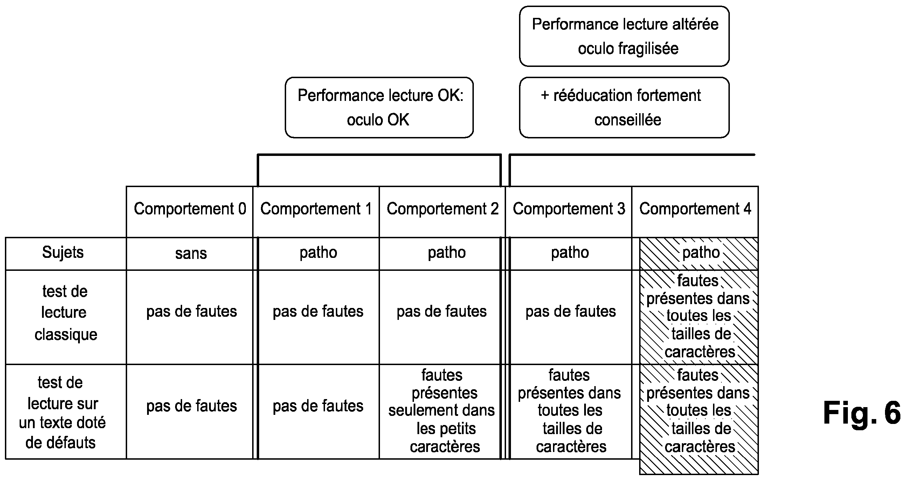

En se référant à la figure 6, les résultats du test de lecture sur un texte comprenant des défauts peuvent être présentés sous la forme de plusieurs catégories, chacune étant représentative du nombre et de l'emplacement des fautes de lecture dans le texte lu. L'exemple de tableau proposé à la figure 6 présente simultanément des résultats à un test de lecture classique et à un test de lecture sur un texte comprenant des défauts. Sur cet exemple, on distingue par exemple, une première catégorie pour laquelle l'individu ne commet aucune faute de lecture aux deux tests, laissant à penser que l'individu ne possède pas de scotome. Une deuxième catégorie correspond à une absence de faute de lecture, alors que l'individu possède un scotome. Une troisième catégorie correspond à aucune faute au test de lecture classique et à des fautes seulement présentes dans les petits caractères lors d'un test de lecture sur un texte possédant des défauts. Une quatrième catégorie correspond à aucune faute au test de lecture classique et à des fautes présentes dans toutes les tailles de caractères lors d'un test de lecture sur un texte possédant des défauts. Une cinquième catégorie correspond à des fautes de lecture dans toutes les tailles de caractères lors d'un test de lecture classique et à des fautes dans toutes les tailles de caractères lors d'un test de lecture sur un texte possédant des défauts. L'indice optique, qui peut par exemple être matérialisé par un chiffre et/ou une lettre, est représentatif des catégories précédemment définies. Cet indice reflète les erreurs de lecture commises par l'individu et donc sa qualité de coordination visuo oculomotrice. Selon une variante de réalisation de l'invention, l'indice peut prendre en compte également la distribution des erreurs de lecture du texte, par exemple des erreurs détectées plus au centre que sur les bords des paragraphes. Le comportement correspondant à la quatrième catégorie précédente est typiquement le comportement majeur à trouver en clinique. L'individu ne réalise pas de fautes avec le test classique, alors qu'un réel problème oculomoteur est présent au test de lecture sur un texte possédant des défauts. Lors d'un test réalisé avec 70 individus, 36% des individus étaient répartis dans cette quatrième catégorie, et 21% dans la cinquième catégorie. Les comportements des quatrième et cinquième catégories nécessitent une prise en charge spécifique, du point de vue oculomoteur en plus d'une prise en charge sensorielle, en termes d'acuité visuelle et de sensibilité aux contrastes.

Le type de comportement est indépendant de l'acuité visuelle et de la localisation de la fixation excentrée, mais est dépendant de la valeur de l'instabilité de fixation.

F- une étape de sélection de moyens d'aide visuelle les plus appropriés en fonction de l'indicateur optique déterminé.

Pour la sélection de l'aide visuelle adaptée, il est important de déterminer les caractéristiques de la vision de l'individu liées à la lecture d'un texte qui dépendent principalement, outre de ses capacités d'identification, de son acuité visuelle, des capacités d'oculomotricité et de l'interaction entre les deux. Le choix de l'aide visuelle la plus adaptée va s'effectuer en fonction de ces 3 caractéristiques.

Le test d'acuité dit dynamique, évaluant la capacité du porteur à mouvoir son scotome lors de l'exploration du texte, permet de déterminer plus efficacement l'acuité en lecture du porteur et d'obtenir une valeur plus précise du grossissement utile de l'équipement d'aide à la vision adapté à l'individu. Si ces capacités sont altérées, le grossissement de l'équipement d'aide à la vision adapté à l'individu devra être augmenté en conséquence. Ce test peut être réalisé avec un support texte ou bien avec des moyens informatiques programmés du type ordinateur, Smartphone, tablette.

Le test d'acuité visuelle est par exemple réalisé par l'affichage de douze lignes de lettres. Chaque ligne de lettre est composée de cinq lettres majuscules. Les lettres utilisées sont les lettres appelées lettres de Sloan, c'est-à-dire S, O, C, D, K, V, R, H, N, Z, comme cela est décrit dans l'ouvrage « Borish's Clinical Refraction », de William J. Benjamin, publié en 2006 par Butterworth-Heinemann/Elsevier. Ces lettres sont en effet facilement reconnaissables. Dans une langue différente du français, et notamment dans une langue utilisant un alphabet différent, on peut également prendre toutes les lettres de l'alphabet ou un groupe de lettres différent.

Sur chaque ligne, la taille des lettres correspond à un angle visuel de discrimination, c'est-à-dire à une acuité visuelle déterminée. La taille des lettres diminue de la première à la dernière ligne.

La taille des lettres est par exemple calibrée pour une distance de lecture de 40 cm. La plage d'acuité visuelle testée dépend ici en partie des caractéristiques de résolution de l'écran tactile utilisé. Il est possible avec les écrans actuels d'afficher des lignes de lettres permettant de tester les acuités visuelles comprises entre 5/10 à 1/25. De préférence, le pas d'acuité visuelle entre chaque ligne est constant, ce qui permet une mesure régulière et fine quelle que soit la plage d'acuités visuelles testée. Pour une ligne donnée, l'espacement entre deux lettres est égal à la taille de la lettre de cette ligne.

Si le test est constitué de phrases, les mots des phrases sélectionnées sont de préférence sélectionnés pour être des mots simples à comprendre. Ces phrases proviennent par exemple d'un texte bien connu du type contes ou fables. Les mots composants ces phrases peuvent également être choisis en fonction de leur occurrence dans la langue considérée. Ces occurrences sont déterminées par des études scientifiques. En français, on trouve les résultats d'une telle étude à l'adresse internet suivante : http://www.lexique.org. Il s'agit de préférence des mots les plus fréquents dans la langue considérée. Chaque phrase comprend de préférence entre 10 et 15 mots présentant une distribution homogène de mots courts, comportant 2 lettres ou moins, de mots de longueurs moyennes, comportant entre 3 et 5 lettres et de mots longs, comportant plus de 5 lettres.

Le test d'oculomotricité permet de déterminer si les yeux du porteur se dirigent avec la même agilité dans toutes les directions. Pour cela, on peut utiliser un test de lecture d'un texte ou de suivi d'une cible. Le test d'oculomotricité permet aussi de déterminer la stabilité de la fixation oculaire. Une cible présentant par exemple la forme d'une croix et de dimensions et contrastes adaptés à l'acuité visuelle du porteur est affichée au centre de l'écran. Une caméra enregistre et suit les mouvements du centre de l'œil, et plus particulièrement le reflet cornéen lumineux provoqué par la présence d'une lumière ponctuelle, par exemple, une diode électroluminescente, dirigée sur l'œil selon une technique de suivi du regard connue en soi. La qualité de la fixation oculaire est évaluée en demandant à la personne de fixer le centre de croix pendant 30 secondes. Une caméra enregistre les variations de position du reflet cornéen durant le test. On détermine ainsi des mouvements continus ou discontinus de l'œil. La mesure peut être effectuée en vision monoculaire ou binoculaire.

L'évaluation de la qualité oculomotrice a un impact dans le choix de l'aide, et plus spécifiquement concernant le champ visuel associé à l'équipement. Un porteur avec une grande instabilité de fixation oculaire aura un meilleur confort et une meilleure performance avec un équipement présentant un grand champ de vision pour l'individu. Un équipement du type télé-agrandisseur serait par exemple dans un tel cas préférable à un équipement du type système de Galilée car présentant un champ de vision bien plus étendu.

Dans notre cas, il s'agit par le biais d'un test d'acuité spécifique, comme par exemple un test de lecture classique de déterminer les caractéristiques de la vision de l'individu liées à la lecture en prenant en compte son acuité visuelle et ses capacités oculomotrices.

Le champ de vision peut être limité par la présence d'une zone « aveugle » appelée scotome. Des personnes malvoyantes présentant par exemple un tel scotome dans le champ de vision peuvent fréquemment présenter des défauts de coordination qui les mènent à interpréter et aussi une stabilité oculaire non régulière car ils vont chercher à utiliser à la fois la vision périphérique et des mouvements répétés des yeux dans plusieurs directions (quasi horizontale par exemple) pour élargir le champ de vision du mot. Cette caractéristique liée à la vision de l'individu joue un rôle important dans le choix de l'équipement d'aide à la vision.

Au préalable l'utilisateur pourra lire une série homogène et ordonnés de texte pour constituer une phase d'entraînement et de mise en confiance de l'individu. Elle permet de vérifier que l'individu a bien compris les instructions de l'opérateur.

Puis il s'agit d'insérer, de manière prédéterminée dans la série des mots affichés, des lettres additionnelles ou d'en supprimer ou bien encore de créer des espacements de sorte à mettre l'individu en échec par rapport un apprentissage automatique ou lecture interprétée. Puis de détecter les défauts assimilés ou non détectés par l'individu lors de la lecture.

Ce test permet de manière simple et ergonomique de caractériser la qualité de la coordination de lecture de l'individu de déterminer un indicateur de lecture en prenant en compte à la fois le test d'acuité et le test d'oculomotricité pour l'optimisation du choix de l'aide visuel.

La présence d'une instabilité de fixation est un indicateur de baisse de performance lors d'une phase de lecture ou d'une tâche d'exploration, dans la lecture mais aussi dans d'autres activités d'explorations.

Dans le cadre de la prise en charge des personnes avec scotomes centraux, la détermination et l'adaptation des aides est différente selon la présence de difficultés oculomotrices :
S'il y a présence de fragilités oculomotrices :
   - on privilégie une aide visuelle à grand champ. Il y a un lien entre le degré de l'instabilité de fixation et la taille du champ de l'aide à proposer pour optimiser les performances. Le confort visuel et la performance/efficacité de lecture sont plus importants. Un système de Galilée (petit champ) est à déconseiller par rapport à une loupe électronique dont le champ de projection de l'image est plus grand.
   - on oriente vers une rééducation visuelle pour améliorer la stabilité de l'œil. Il a été constaté une diminution du nombre de fautes au test de lecture classique, après une dizaine de séances de rééducation oculomotrice. Une amélioration de la stabilité de fixation diminue le nombre de fautes réalisée à ce test.
   - Dans le cas d'adaptation de verres progressifs définis dans la norme ISO 13666:2012, on peut concevoir un design de verres progressifs selon le degré d'instabilité de l'œil. On peut imaginer une largeur du couloir de vision nette adaptée à la valeur de l'instabilité de fixation.
De façon non exhaustive, les moyens d'aide visuelle permettant de pallier la présence d'une anomalie visuelle et oculomotrice liée à la présence de scotomes, sont les suivants,
   - des loupes,
   - des lunettes microscopiques,
   - des systèmes télescopiques de type Galilée ou Kepler,
   - des aides électroniques portables,
   - des aides électroniques de type télé-agrandisseurs,
   - des lampes,
   - des filtres,
   - des lunettes équipées de moyens de réalité augmentée,
   - des verres progressifs à forte addition,
   - une rééducation oculomotrice et visuo oculomotrice.

## Revendications

1. Procédé de détermination d'une aide visuelle suite au comportement d'un individu lors d'une phase de lecture spécifique, comprenant les étapes suivantes,
- une étape de soumission à l'individu d'un test spécifique de lecture d'un support comportant un texte comprenant une succession de mots usuels et dans lesquelles ont été introduits des défauts entre lesdits mots à des emplacements prédéterminés, lesdits défauts introduisant des perturbations aptes à rendre le texte dépourvu de sens,
- une étape de lecture à voix haute dudit texte par l'individu,
- une étape d'attribution d'un indicateur optique lié aux erreurs de lecture relevées et permettant de caractériser la qualité de la lecture,
- une étape de sélection des moyens d'aide visuelle les plus appropriés en fonction de l'indicateur optique déterminé.

2. Procédé de détermination selon la revendication 1, **caractérisé en ce que** chaque défaut de texte est à choisir parmi un ajout d'au moins un blanc, un ajout d'au moins une lettre, une suppression d'au moins un blanc et une suppression d'au moins une lettre.

3. Procédé de détermination selon l'une quelconque des revendications 1 ou 2, **caractérisé en ce que** le texte comprend plusieurs paragraphes comprenant des lettres d'une taille spécifique et un espacement spécifique entre les lettres de chaque mot et entre les mots.

4. Procédé de détermination selon la revendication 3, **caractérisé en ce que** tous les paragraphes du support ont des tailles de lettres différentes, et des espacements différents entre les lettres de chaque mot et entre les mots.

5. Procédé de détermination selon l'une quelconque des revendications 3 ou 4, **caractérisé en ce que** l'indicateur optique est fonction des caractéristiques dimensionnelles des paragraphes dans lesquels ont été commises des erreurs de lecture.

6. Procédé de détermination selon l'une quelconque des revendications 1 à 5, **caractérisé en ce que** le support de lecture comprend des séquences de motifs identifiables, **caractérisées par** une succession de groupes de motifs possédant chacun un nombre spécifique de motifs et séparés les uns des autres par des blancs, et **en ce que** l'espacement entre deux motifs successifs dans un groupe est identique pour tous les groupes.

7. Procédé de détermination selon la revendication 6, **caractérisé en ce que** chaque séquence comprend des motifs identiques.

8. Procédé de détermination selon l'une quelconque des revendications 1 à 7, **caractérisé en ce que** l'indicateur optique est apte à révéler une anomalie visuelle constituée par un scotome et/ou une instabilité oculaire.

9. Procédé de détermination selon l'une quelconque des revendications 1 à 8, **caractérisé en ce que** l'individu adopte une posture naturelle durant l'étape de lecture du support.

10. Procédé de détermination selon l'une quelconque des revendications 1 à 9, **caractérisé en ce que** le support est un écran placé à une distance prédéterminée de l'individu.

11. Procédé de détermination selon l'une quelconque des revendications 1 à 10, **caractérisé en ce qu'**il comprend une étape préalable de lecture par l'individu d'un texte ayant un sens et utilisant des mots usuels, et **en ce que** les erreurs de lecture relevées lors de cette étape, vont conditionner les conditions de réalisation de l'étape de soumission du test spécifique.

12. Procédé de détermination selon l'une quelconque des revendications 1 à 11, **caractérisé en ce qu'**il comprend une étape de réalisation d'une table de critères optiques, comprenant au moins trois catégories distinctes et identifiables, une première catégorie correspondant à une vision normale, une deuxième catégorie pour laquelle la vision présente quelques anomalies et une troisième catégorie pour laquelle la vision présente un nombre important d'anomalies.

13. Procédé de détermination la revendication 12, **caractérisé en ce qu'**il comprend une étape d'enregistrement de la table de critères optiques.

14. Procédé de détermination selon l'une quelconque des revendications 1 à 13, **caractérisé en ce qu'**il comprend une étape d'élaboration d'une base de données établissant une corrélation entre l'indice optique et des moyens d'aide à une correction visuelle les plus adaptés.

15. Procédé de détermination selon l'une quelconque des revendications 1 à 14, **caractérisé en ce que** les moyens d'aide à la vision sont à choisir parmi des loupes, des lunettes microscopiques, des systèmes télescopiques de type Galilée ou Kepler, des aides électroniques portables, des aides électroniques de type télé-agrandisseurs, des lampes, des filtres, des lunettes équipées de moyens de réalité augmentée, des verres progressifs à forte addition et des séances de rééducation oculomotrice.

16. Dispositif pour la réalisation d'un procédé de détermination conforme à l'une quelconque des revendications 1 à 15, étant constitué par un terminal numérique mobile, possédant un logiciel adapté et un accès à des bases de données enregistrées.

17. Dispositif selon la revendication 16, le terminal numérique mobile étant une tablette tactile, apte à faire apparaitre le support de lecture et à enregistrer la voix de l'individu, et en ce que ladite tablette possède un logiciel de détermination de l'indice optique ainsi qu'une table de correspondance entre ledit indice optique et l'anomalie visuelle correspondante, ladite tablette possédant en outre un accès à des bases de données établissant une corrélation entre l'indice optique et des moyens d'aide visuelle les plus adaptés.

## Patentansprüche

1. Verfahren zur Bestimmung einer Sehhilfe nach dem Verhalten einer Person bei einer bestimmten Lesephase, umfassend die folgenden Schritte,
- einen Schritt des Unterziehens der Person einem bestimmten Test des Lesens eines Trägers, der einen Text mit einer Aufeinanderfolge von gebräuchlichen Wörtern aufweist, und in welche zwischen den Wörtern an vorbestimmten Stellen Fehler eingefügt wurden, wobei die Fehler Störungen einfügen, die geeignet sind, den Text sinnfrei zu machen,
- einen Schritt des lauten Lesens des Textes durch die Person,
- einen Schritt des Zuweisens eines optischen Indikators, der mit den festgestellten Lesefehlern in Verbindung steht und es gestattet, die Qualität des Lesens zu charakterisieren,
- einen Schritt des Auswählens der geeignetsten Seehilfsmittel in Abhängigkeit vom bestimmten optischen Indikator.

2. Bestimmungsverfahren nach Anspruch 1, **dadurch gekennzeichnet, dass** jeder Textfehler aus einer Hinzufügung mindestens eines Leerzeichens, einer Hinzufügung mindestens eines Buchstabens, einer Löschung mindestens eines Leerzeichens und einer Löschung mindestens eines Buchstabens zu wählen ist.

3. Bestimmungsverfahren nach einem der Ansprüche 1 oder 2, **dadurch gekennzeichnet, dass** der Text mehrere Absätze umfasst, die Buchstaben einer bestimmten Größe und einen bestimmten Abstand zwischen den Buchstaben jedes Worts und zwischen den Wörtern umfassen.

4. Bestimmungsverfahren nach Anspruch 3, **dadurch gekennzeichnet, dass** alle Absätze des Trägers verschiedene Buchstabengrößen und verschiedene Abstände zwischen den Buchstaben jedes Worts und zwischen den Wörtern aufweisen.

5. Bestimmungsverfahren nach einem der Ansprüche 3 oder 4, **dadurch gekennzeichnet, dass** der optische Indikator von den Abmessungscharakteristika der Absätze abhängig ist, in welchen Lesefehler gemacht wurden.

6. Bestimmungsverfahren nach einem der Ansprüche 1 bis 5, **dadurch gekennzeichnet, dass** der Leseträger Sequenzen identifizierbarer Motive umfasst, **gekennzeichnet durch** eine Aufeinanderfolge von Motivgruppen, die jeweils eine bestimmte Anzahl von Motiven aufweisen und durch Leerzeichen voneinander getrennt sind, und **dadurch, dass** der Abstand zwischen zwei aufeinanderfolgenden Motiven in einer Gruppe für alle Gruppen identisch ist.

7. Bestimmungsverfahren nach Anspruch 6, **dadurch gekennzeichnet, dass** jede Sequenz identische Motive umfasst.

8. Bestimmungsverfahren nach einem der Ansprüche 1 bis 7, **dadurch gekennzeichnet, dass** der optische Indikator geeignet ist, eine Sehstörung festzustellen, die von einem Skotom und/oder einer Augeninstabilität gebildet ist.

9. Bestimmungsverfahren nach einem der Ansprüche 1 bis 8, **dadurch gekennzeichnet, dass** die Person während des Schritts des Lesens des Trägers eine natürliche Haltung einnimmt.

10. Bestimmungsverfahren nach einem der Ansprüche 1 bis 9, **dadurch gekennzeichnet, dass** der Träger eine Bildfläche ist, die in einem vorbestimmten Abstand zur Person platziert ist.

11. Bestimmungsverfahren nach einem der Ansprüche 1 bis 10, **dadurch gekennzeichnet, dass** es einen vorhergehenden Schritt des Lesens, durch die Person, eines Texts, der eine Richtung aufweist und gebräuchliche Wörter verwendet, umfasst, und dadurch, dass die Lesefehler, die bei diesem Schritt festgestellt werden, die Durchführungsbedingungen des Schritts des Unterziehens des bestimmten Tests beeinflussen.

12. Bestimmungsverfahren nach einem der Ansprüche 1 bis 11, **dadurch gekennzeichnet, dass** es einen Schritt des Erstellens einer Tabelle optischer Kriterien umfasst, die mindestens drei verschiedene und identifizierbare Kategorien umfassen, wobei eine erste Kategorie einer normalen Sicht entspricht, eine zweite Kategorie, bei der sie Sicht einige Störungen aufweist, und eine dritte Kategorie, bei der die Sicht eine große Anzahl von Störungen aufweist.

13. Bestimmungsverfahren nach Anspruch 12, **dadurch gekennzeichnet, dass** es einen Schritt des Speicherns der Tabelle optischer Kriterien umfasst.

14. Bestimmungsverfahren nach einem der Ansprüche 1 bis 13, **dadurch gekennzeichnet, dass** es einen Schritt des Erstellens einer Datenbank umfasst, die eine Korrelation zwischen dem optischen Index und geeignetsten Hilfsmitteln zu einer Sehkorrektur herstellt.

15. Bestimmungsverfahren nach einem der Ansprüche 1 bis 14, **dadurch gekennzeichnet, dass** die Sehhilfsmittel aus Lupen, Mikroskopbrillen, Teleskopsystemen vom Gallileo- oder Keplertyp, tragbaren elektronischen Hilfen, elektronischen Hilfen vom Typ Bildschirmvergrößerungsgerät, Lampen, Filtern, Brillen, die mit Augmented-Reality-Mittel ausgerüstet sind, progressiven Gläsern mit hoher Addition und Sitzungen zur okulomotorischen Rehabilitation zu wählen sind.

16. Vorrichtung zur Durchführung eines Bestimmungsverfahrens nach einem der Ansprüche 1 bis 15, die von einem mobilen digitalen Endgerät gebildet ist, das eine geeignete Software und einen Zugang zu gespeicherten Datenbanken aufweist.

17. Vorrichtung nach Anspruch 16, wobei das mobile digitale Endgerät ein Berührungstablet ist, das geeignet ist, den Leseträger erscheinen zu lassen und die Stimme der Person aufzunehmen, und dadurch, dass das Tablet eine Software zur Bestimmung des optischen Index sowie eine Tabelle der Entsprechung zwischen dem optischen Index und der entsprechenden Sehstörung aufweist, wobei das Tablet ferner einen Zugang zu Datenbanken aufweist, die eine Korrelation zwischen dem optischen Index und geeignetsten Sehhilfsmitteln herstellen.

## Claims

1. Method for determining a visual aid depending on the behavior of an individual during a specific reading phase, comprising the following steps,
- a step in which the individual is made to take a specific test in which a medium including a text comprising a succession of commonplace words and into which defects have been introduced between said words in predetermined locations is read, said defects introducing disruptions apt to make the text devoid of sense,
- a step in which the individual reads said text out loud,
- a step in which an optical indicator related to noted reading errors and allowing the quality of the reading to be characterized is attributed,
- a step of selecting the most appropriate visual aiding means depending on the determined optical indicator.

2. Determining method according to Claim 1, **characterized in that** each text defect is chosen from an addition of at least one space, an addition of at least one letter, a removal of at least one space and a removal of at least one letter.

3. Determining method according to either one of Claims 1 and 2, **characterized in that** the text comprises a plurality of paragraphs comprising letters of a specific size and a specific spacing between the letters of each word and between the words.

4. Determining method according to Claim 3, **characterized in that** all the paragraphs of the medium have different sizes of letters, and different spacings between the letters of each word and between the words.

5. Determining method according to either one of Claims 3 and 4, **characterized in that** the optical indicator is dependent on dimensional characteristics of the paragraphs in which reading errors were made.

6. Determining method according to any one of Claims 1 to 5, **characterized in that** the reading medium comprises sequences of identifiable patterns, said sequences being **characterized by** a succession of groups of patterns each possessing a specific number of patterns and separated from one another by spaces, and **in that** the spacing between two successive patterns in a group is identical for all the groups.

7. Determining method according to Claim 6, **characterized in that** each sequence comprises identical patterns.

8. Determining method according to any one of Claims 1 to 7, **characterized in that** the optical indicator is apt to reveal a visual anomaly consisting of a scotoma and/or an ocular instability.

9. Determining method according to any one of Claims 1 to 8, **characterized in that** the individual adopts a natural posture during the step of reading the medium.

10. Determining method according to any one of Claims 1 to 9, **characterized in that** the medium is a screen placed at a predetermined distance from the individual.

11. Determining method according to any one of Claims 1 to 10, **characterized in that** it comprises a prior step in which the individual reads a text having a meaning and using commonplace words, and **in that** the conditions under which the step in which the specific test is taken are dependent on reading errors noted in said prior step.

12. Determining method according to any one of Claims 1 to 11, **characterized in that** it comprises a step of producing a table of optical criteria, comprising at least three distinct and identifiable categories, a first category corresponding to normal vision, a second category for which the vision presents a few anomalies and a third category for which the vision presents a large number of anomalies.

13. Determining method according to Claim 12, **characterized in that** it comprises a step of recording the table of optical criteria.

14. Determining method according to any one of Claims 1 to 13, **characterized in that** it comprises a step of building a database establishing a correlation between the optical index and the most suitable means for aiding with a visual correction.

15. Determining method according to any one of Claims 1 to 14, **characterized in that** the means for aiding with vision are chosen from magnifying glasses, microscopic spectacles, telescopic systems of the Galilean or Keplerian type, portable electronic aids, electronic aids of the TV-video-magnifier type, lamps, filters, spectacles equipped with augmented reality means, progressive lenses of high addition and oculomotile re-education sessions.

16. Device for carrying out a determining method according to any one of Claims 1 to 15, consisting of a mobile digital terminal possessing a suitable software package and an access to recorded databases.

17. Device according to Claim 16, the mobile digital terminal being a touch tablet able to make the reading medium appear and to record the voice of the individual, and in that said tablet possesses an optical-index-determining software package, and a lookup table describing the correspondence between said optical index and the corresponding visual anomaly, said tablet furthermore possessing an access to databases establishing a correlation between the optical index and the most suitable visual aiding means.
